(19) Europäisches Patentamt / European Patent Office / Office européen des brevets

(11) **EP 4 424 333 B1**

(12) **EUROPEAN PATENT SPECIFICATION**

(45) Date of publication and mention
of the grant of the patent:
**18.03.2026   Bulletin 2026/12**

(21) Application number: **22897615.5**

(22) Date of filing: **09.11.2022**

(51) International Patent Classification (IPC):
***A61L 27/48*** *(2006.01)*

(52) Cooperative Patent Classification (CPC):
(C-Sets available)
**C08B 37/0072; A61L 27/12; A61L 27/46;
A61L 27/48; A61L 27/52; C08G 63/912;
C08J 3/075;** A61L 2400/06; C08J 2305/08;
C08J 2467/04; C08K 5/098; C08K 2003/265;
C08K 2003/325                    (Cont.)

(86) International application number:
**PCT/CN2022/130905**

(87) International publication number:
**WO 2023/093532 (01.06.2023 Gazette 2023/22)**

(54) **COMPOSITE GEL, PREPARATION METHOD, AND APPLICATION**

VERBUNDGEL, HERSTELLUNGSVERFAHREN UND ANWENDUNG

GEL COMPOSITE, PROCÉDÉ DE PRÉPARATION ET APPLICATION

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **29.11.2021   CN 202111431502**

(43) Date of publication of application:
**04.09.2024   Bulletin 2024/36**

(73) Proprietor: **Imeik Technology Development Co.,
Ltd.
Beijing 100022 (CN)**

(72) Inventors:
• **LI, Ruizhi
Beijing 100022 (CN)**
• **GU, Shiwei
Beijing 100022 (CN)**
• **ZHANG, Kun
Beijing 100022 (CN)**

(74) Representative: **Viering, Jentschura & Partner
mbB
Patent- und Rechtsanwälte
Am Brauhaus 8
01099 Dresden (DE)**

(56) References cited:
CN-A- 101 590 388     CN-A- 102 772 827
CN-A- 102 911 380     CN-A- 104 258 470
CN-A- 105 107 026     CN-A- 105 126 166
CN-A- 109 172 874     CN-A- 109 621 003
CN-A- 110 559 489     CN-A- 113 286 623
KR-A- 20110 137 907

**(Cont. next page)**

(52) Cooperative Patent Classification (CPC): (Cont.)

C-Sets
**A61L 27/48, C08L 5/08;**
**A61L 27/48, C08L 67/04;**
**C08L 5/08, C08L 67/04;**
**C08L 5/08, C08L 67/04, C08K 3/32**

## Description

## Technical Field

The present disclosure relates to the technical field of biomedical materials, and in particular, to a composite gel, and a preparation method and an application thereof.

## Background

Polylactic Acid (PLA) is also referred to as polylactide, and is a polyester polymer obtained through polymerization by using lactic acid as a main material. The Polylactic Acid is used in combination with Hydroxyapatite (HAP) due to the characteristics of being good in biocompatibility and biodegradability, easy to process, etc., so as to improve the mechanical performance and biological performance of the Hydroxyapatite. Currently, the injectable products that promote tissue regeneration have been widely accepted worldwide, and the Polylactic Acid acts as a main material in this kind of products. After entering the skin of a human body, the Polylactic Acid can stimulate the regeneration of tissue of the human body itself. Through the regeneration of the tissue of the human body itself, the effect of structure remodeling and volume filling is realized, so as to achieve an anti-aging purpose. Tissue that can be regenerated after being stimulated mainly includes fibroblasts and collagen. The Polylactic Acid can stimulate its own fibroblasts to secrete collagen, so as to make filling and repairing effects more natural, and simultaneously improve skin glossiness, thereby achieving the purpose of overall facial rejuvenation. However, the natural defect of the Polylactic Acid is that the degradation of the Polylactic Acid in the human body produces lactic acid, which is metabolized slowly by the human body, and the aggregation of the large amount of lactic acid tends to cause aseptic inflammation, leading to a series of adverse reactions. Therefore, when the Polylactic Acid needs to be used, an alkaline neutralizer is added to reduce the aseptic inflammation due to localized over-acidity. Therefore, the alkaline Hydroxyapatite with good biocompatibility has become an excellent choice as an alkaline neutralizer for the Polylactic Acid.

A composite material formed by the Polylactic Acid and an inorganic material has been applied to biomaterials. For example, CN106139256A disclosed a Polylactic Acid/nano β-tricalcium phosphate composite porous scaffold, and CN111905151A disclosed a mesoporous bioactive glass/Polylactic Acid-hydroxyacetic acid copolymer composite microsphere.

In particular, a composite material of the Hydroxyapatite and the Polylactic Acid has been widely used in the field such as orthopedics. Huang Zhihuan et, al. (Journal of Composite Materials, 2021, 38: 749-760), in the study of nano HAP-poly (L-lactide) (PLLA) composites, found that compressive yield strength and tensile strength of PLLA added with HAP respectively increased by 9.4% and 6.6% compared with a pure PLLA material, initial decomposition temperatures increased by 7.4% compared with pure PLLA, and a degree of crystallinity increased by 6.7%. Therefore, the PLLA material added with HAP has significant advantages over the pure PLLA material in terms of material science properties. In addition, the HAP may reduce inflammation. Compared with the PLA material added with HAP, the pure PLA shows a stronger inflammatory response, while the HAP-PLA composite material only shows slight inflammatory response in vivo. It may be demonstrated that the addition of HAP can neutralize acidic substances due to PLA degradation and reduce aseptic inflammation due to acidity.

There have been reports of binary composites of Hydroxyapatite and Polylactic Acid, and ternary composites of Hydroxyapatite, PolylacticAcid, and other polymers, or composite microspheres at home and abroad. Dong Yan (Additive Manufacturing, 2020; 34:101305) et, al. have prepared Polylactic Acid and nanoscale Hydroxyapatite blended microspheres, which have the minimum particle size being approximately 100 μm, and are mainly used in research such as microcarriers. Patents such as a method for preparing nano Hydroxyapatite/Polylactic Acid composite microspheres (CN101590388A), a Polylactic Acid porous microsphere carrying Hydroxyapatite and a preparation method thereof (CN103868658B), a Polylactic Acid/Hydroxyapatite whisker composite porous scaffold for bone tissue and a preparation method thereof (CN105797215A), a Polylactic Acid-Hydroxyapatite micron nano multi-level structure composite microsphere material and an application thereof (CN 109749119 A), a controllable preparation method for a Polylactic Acid-Hydroxyapatite composite microsphere having a porous surface structure and an application thereof (CN107519536A), a method for preparing Hydroxyapatite/modified Polylactic Acid composite microspheres (CN109350768A), and a method for preparing Hydroxyapatite/ Polylactic Acid/chitosan composite microspheres (CN102489231B) are mainly applied to the repairing, filling and drug-carrying of bone defects, and have not been applied to the field of tissue filling.

CN 109 621 003 A discloses a preparing method of injectable sodium hyaluronate gel containing microballoons. The preparing method comprises the following steps of dissolving sodium hyaluronate into purewater to obtain a solution A; adding a microballoon material into the solution A, and conducting uniform stirring to obtain a homodisperse suspension liquid B; adjusting the PH value of the suspension liquid B to obtain a suspension liquid C; adding a crosslinking agent into the suspension liquid C, conducting uniform stirring to obtain a homodisperse suspension liquid D, after a reaction is carried out for a certain period of time, crosslinking sodium hyaluronate in the suspension liquid D to form colloid E containing the homodisperse microballoon material; smashing the colloid E to obtain gel particles F, and obtaining gel particles G through screens with different mesh numbers; removing soluble impurities and crosslinking agents in the gel particles G in a repeated soaking or dialytic mode to obtain gel particles H; utilizing a PBS buffering solution for balancing for repeatedly soaking the gel particles H to obtain gel particles I; obtaining the sodium hyaluronate gel through the procedures of semi-dehydration, filling and sterilization. By means of the preparing method, the problem that the microballoon material is deposited and gets caked is effectively solved.

## Summary

The present disclosure is intended to provide a composite gel, and a preparation method and an application thereof. The composite gel integrates the advantages of three materials of hyaluronic acid, Polylactic Acid, and an inorganic material, and may be used for filling and repairing biological tissue; the crosslinking comprises crosslinking of Polylactic Acid in the biphasic microspheres with the hyaluronic acid; the Polylactic Acid is micron-sized; the inorganic material is selected from one or more of calcium carbonate, calcium phosphate, and calcium tartrate; the inorganic material is a nano inorganic material.

The present disclosure provides the following technical solutions.

A composite gel is prepared by crosslinking biphasic microspheres with hyaluronic acid. The biphasic microspheres are Polylactic Acid/inorganic material biphasic microspheres.

Further, the calcium phosphate is Hydroxyapatite.

In an implementation solution, the inorganic material is nano Hydroxyapatite calcium.

Further, the hyaluronic acid is cross-linked hyaluronic acid; further, the cross-linked hyaluronic acid is prepared through a crosslinking agent; and the crosslinking agent is selected from one or more of dialdehyde, disulfide, Polyethylene Glycol (PEG) crosslinking agent, divinyl sulphone, diglycidyl ether, di-epoxide, diamine and polyamine.

Further, the crosslinking agent is selected from bis(carbodiimide), aliphatic diamine, ethylenediamine, hexamethylene diamine, endogenous polyamine (spermine or spermidine), Pentaerythritol Tetraglycidyl Ether (PETGE), Divinyl Sulfone (DVS), 1,4-butanediol diglycidyl ether (BDDE), 1,2-bis(2,3-epoxypropoxyl)ethylene (EGDGE), 1, 2, 7, 8-diepoxyoctane (DEO), (phenylene bis-(ethyl)-carbodiimide and 1,6-hexamethylene bis(ethyl carbodiimide), Adipic Dihydrazide (ADH), bis(sulfosuccinimide)octanedioic acid ester (BS), hexamethylenediamine (HMDA), 1-(2,3-epoxypropyl)-2,3-epoxycyclohexane, 1,4-bis(2,3-epoxypropoxyl)butane, 1,4-bis(glycidyl ether)oxybutane, 1-(2,3-epoxypropyl)-2,3-epoxycyclohexane, 1,3-butadiene diepoxide, 1, 2, 7, 8-diepoxyoctane, and 1,5-hexadiene diepoxide. Preferably, the crosslinking agent is diamine or polyamine, and a reaction process may refer to patents CN99813143.1, US9907739, CN202111326226.7, etc.

Further, the crosslinking of the Polylactic Acid with the hyaluronic acid includes any one of physical electrostatic crosslinking and chemical crosslinking. The chemical crosslinking includes reacting the Polylactic Acid in the biphasic microspheres with the hyaluronic acid to form an amide bond crosslinking reaction. The physical electrostatic crosslinking includes performing surface charge modification on the biphasic microspheres, and then forming electrostatic crosslinking with the hyaluronic acid. Further, the chemical crosslinking includes performing amino modification on the Polylactic Acid in the biphasic microspheres, and then performing a reaction with the hyaluronic acid to form the amide bond crosslinking reaction. The physical electrostatic crosslinking includes performing surface charge modification on the biphasic microspheres, and then forming electrostatic crosslinking with cross-linked hyaluronic acid having positive ions.

An implementation solution provides a composite gel, which is prepared by crosslinking hyaluronic acid with Polylactic Acid biphasic microspheres in which nanoscale Hydroxyapatite is dispersed.

A method for preparing a composite gel includes crosslinking biphasic microspheres with hyaluronic acid to form a composite gel (triphasic composite gel). The biphasic microspheres are Polylactic Acid/inorganic material biphasic microspheres.

Further, crosslinking the biphasic microspheres with the hyaluronic acid includes crosslinking of Polylactic Acid in the biphasic microspheres with the hyaluronic acid.

Further, the hyaluronic acid is non-cross-linked hyaluronic acid or cross-linked hyaluronic acid. In an implementation solution, the hyaluronic acid is cross-linked hyaluronic acid, is preferably diamine or polyamine cross-linked hyaluronic acid, and is more preferably endogenous polyamine cross-linked hyaluronic acid.

In an implementation solution, in the Hydroxyapatite dispersed Polylactic Acid biphasic microspheres, the mass of the Hydroxyapatite accounts for 0.1-50% of the total mass of the microspheres. The rest of the microspheres except the Hydroxyapatite are the Polylactic Acid.

In an implementation solution, cross-linked hyaluronic acid hydrogel is prepared by using polyamine as a crosslinking agent, and preferably, the mass of the polyamine crosslinking agent accounts for 0.5-20% of the total mass of the hyaluronic acid. In the hydrogel, a mass concentration of the hyaluronic acid ranges from 1 to 35 mg/mL.

In an implementation solution, the hyaluronic acid is prepared by a microbial fermentation method; and preferably, the molecular weight of the hyaluronic acid ranges from 100 KDa to 3,000 KDa, and preferably ranges from 500 KDa to 1,500 KDa. The hyaluronic acid obtained through microbial fermentation is non-animal derived hyaluronic acid, which has a more uniform molecular weight distribution.

In an implementation solution, the crosslinking is solid phase crosslinking; and further, the solid phase crosslinking includes any one of physical electrostatic crosslinking and chemical crosslinking.

In an implementation solution, the chemical crosslinking includes performing amino modification on the Polylactic Acid in the biphasic microspheres, and then reacting the modified biphasic microspheres with the hyaluronic acid to form amide bond crosslinking; further, the amino modification includes reacting the biphasic microspheres with an amine compound; and further the amide bond crosslinking includes performing a coupling reaction on the modified biphasic microspheres and the hyaluronic acid under the presence of a carbodiimide activating agent.

In an implementation solution, the amine compound contains diamine, polyamine, or a polyamino compound. Preferably, the diamine includes any one of aliphatic diamine, aromatic diamine, and heteroatom diamine, for example, but is not limited to, aliphatic diamine, ethylenediamine, and hexamethylene diamine. The polyamine includes any one of aliphatic polyamine, aromatic polyamine, and heteroatom polyamine, for example, but is not limited to, endogenous polyamine such as spermidine and spermine. The polyamino compound includes any one of bis-terminal aminated PEG and terminal aminated multiarm PEG.

Further, the carbodiimide activating agent includes one or more of 1-ethyl-3-(3-dimethylaminopropyl)carbodiimide(EDC), 1-(3-dimethylaminopropyl)-3-ethyl-carbodiimide, 1-cyclohexyl-3-(2-morpholinoethyl)carbodiimide, 1,3-bis [di(methoxymethyl)methyl]carbodiimide, and salts thereof.

In an implementation solution, the preparation method further includes performing combined use on an auxiliary with the carbodiimide activating agent, for example, N-hydroxysuccinimide (NHS), tertiary butanol, etc. The water-soluble carbodiimide activating agent needs to be used in combination with the auxiliary to improve the efficiency of the crosslinking reaction, and an addition amount of the auxiliary is 10-30% of the mass of the carbodiimide.

In an implementation solution, a mass ratio of the biphasic microspheres to the hyaluronic acid in the chemical crosslinking is 1:0.5-20, and is preferably 1:1-10.

An addition amount of the amine compound in the amino modification reaction system is 30-200% of the mass of the biphasic microspheres, and the concentration of the amine compound ranges from 10 to 1,000 mg/mL.

Further, an addition amount of the carbodiimide activating agent is 10-150% of the total mass of the hyaluronic acid.

Further, the concentration of a hyaluronic acid solution is 10-100 mg/mL.

Further, reaction temperatures of the amino modification and the amide bond crosslinking are 5-60°C, and reaction time is 6-24 h. A pH value of the reaction system ranges from 5.0 to 6.0.

In an implementation solution, the microspheres are Hydroxyapatite dispersed Polylactic Acid biphasic microspheres. The chemical crosslinking includes dispersing the Hydroxyapatite dispersed Polylactic Acid biphasic microspheres in water, adding the amine compound (polyamine derivative agent) to regulate a pH value, so as to make carboxyl on the surfaces of the microspheres and the polyamine derivative agent subjected to a surface modification reaction, and modifying aminos on the surfaces; and putting the Hydroxyapatite dispersed Polylactic Acid biphasic microspheres with amino modified surfaces (Polylactic Acid-Hydroxyapatite biphasic microspheres) into a hyaluronic acid solution, regulating the pH value to 5.0-6.0, and at the same time, adding the carbodiimide activating agent and the auxiliary, so as to complete a coupling reaction of the Polylactic Acid-Hydroxyapatite biphasic microspheres. A reaction temperature of surface modification is 5-60°C, and reaction time is 6-24 h; and the temperature of the coupling reaction is 5-60°C, and reaction time is 6-24 h.

The physical electrostatic crosslinking includes performing surface charge modification on the biphasic microspheres to obtain modified biphasic microspheres with surfaces carrying negative ions, and mixing the modified biphasic microspheres and cross-linked hyaluronic acid having positive ions, so as to perform electrostatic crosslinking. Further, the cross-linked hyaluronic acid having positive ions is cross-linked hyaluronic acid obtained through diamine or polyamine crosslinking. Further, performing surface charge modification on the biphasic microspheres includes soaking the biphasic microspheres in an alkaline buffer solution, and obtaining the modified biphasic microspheres through separation.

Further, a mass ratio of the biphasic microspheres to the cross-linked hyaluronic acid in the physical crosslinking is 1:1-50, and is preferably 1:1-20.

Further, the alkaline buffer solution is an alkaline phosphate buffer solution, and the pH of the alkaline phosphate buffer solution preferably ranges from 9.0 to 11.0.

In an implementation solution, the microspheres are Hydroxyapatite dispersed Polylactic Acid biphasic microspheres. The physical crosslinking includes soaking the Hydroxyapatite dispersed Polylactic Acid biphasic microspheres into a PBS with pH being 9.0-11.0 for surface charge modification, to make the surfaces of the microspheres carry negative charges, separating and taking out the biphasic microspheres, and physically mixing same with the cross-linked hyaluronic acid gel using polyamine as the crosslinking agent, so as to cause negative carboxylate ions on the surface of the Polylactic Acid to be subjected to electrostatic crosslinking with positive ammonium ions in the cross-linked hyaluronic acid.

In an implementation solution, the present disclosure further includes a method for preparing Hydroxyapatite dispersed Polylactic Acid biphasic microspheres. The method includes the following steps.

(a) Polylactic Acid (PLA) is dissolved in dichloromethane, and fully stirred, dissolved, and prepared into a solution, and then, insoluble substances are removed through filtration.

(b) Hydroxyapatite is added for ultrasound, uniform stirring is performed to completely disperse the Hydroxyapatite in the solution, so as to form suspension in which the Hydroxyapatite is uniformly dispersed.

(c) A polyvinyl alcohol solution is prepared in proportion to the dichloromethane for stirring, in the meantime, the dichloromethane suspension is added to the system dropwise, and the liquid particles are formed under the action of stirring and shearing as well as the action of emulsification.

(d) Water bath and stirring are performed on the emulsified solution, the dichloromethane is removed through volatilization, and then the obtained solution is washed and filtered.

In a specific implementation solution, in the step (a), preferably, the concentration of the dichloromethane solution of the PLA is 10 mg/mL-80 mg/mL, including, but not limited to, 15 mg/mL, 30 mg/mL, 40 mg/mL, 60 mg/mL and 80 mg/mL.

Preferably, the PLA contains a mixture of one or more of Poly (DL-lactic acid) (PLLA), Poly (DL-lactic acid) (PDLLA), and Poly (D-lactic acid) (PDLA).

Preferably, the molecular weight of the PLA ranges from 10 KDa to 150 KDa.

In a specific implementation solution, in the step (b), preferably, a mass fraction of the Hydroxyapatite is 1%-20% to ensure that the Hydroxyapatite can be completely dispersed into the Polylactic Acid microspheres.

Preferably, ultrasound energy is 1-300 KJ; preferably, the energy is 1-20 KJ; and the ultrasound energy is calculated according to the following formula.

Ultrasound energy (KJ) = ultrasound power (W) $\times$ ultrasound time (s)/1000.

In a specific implementation solution, in the step (c), preferably, the concentration of the polyvinyl alcohol solution is 5 mg/mL-30 mg/mL, an emulsification effect may be guaranteed within the concentration range, and the sizes of the microspheres are controlled.

Preferably, a ratio of the dichloromethane solution to the polyvinyl alcohol solution is 1/2-1/10.

Preferably, a rotary speed of a stirring paddle in a stirring device is 100-600 rpm; and within this rotary speed range, continuous emulsification can be guaranteed to form a desirable emulsion.

In a specific implementation solution, in the step (d), preferably, the temperature of the water bath is 20-37°C, a volatilization rate of the dichloromethane may be controlled within the temperature range, such that a hollow effect of the microspheres and a surface microporous structure are optimized. After the step (d), the Hydroxyapatite dispersed Polylactic Acid biphasic microspheres may be obtained by drying a filter cake obtained. Preferably, the drying process includes two modes: vacuum drying and freeze drying; the vacuum drying is to reduce a water boiling point under an approximate vacuum condition, and volatilize moisture at a low temperature; and the freeze drying method uses an ice crystal sublimation principle, such that internal structures of the microspheres are prevented from being damaged during a solid phase component liquefaction process, and spatial structures of the Polylactic Acid microspheres obtained through preparation can be better maintained.

In an implementation solution, an average elastic modulus of the composite gel of the present disclosure is 400 to 1,000 Pa.

In an implementation, a loss ratio of the elastic modulus of the composite gel of the present disclosure before and after sterilization is lower than 30%, and is preferably lower than 20%. In another aspect, the present disclosure protects the composite gel prepared by the preparation method.

In still another aspect, the present disclosure further protects an application of the composite gel in preparation of drug carriers, tissue fillers, or tissue repairing materials.

Further, the drug carriers may be drug carriers of biological drugs, chemical drugs, or naturally-extracted drugs.

Beneficial effects are as follows:

(1) In the present disclosure, Polylactic Acid-inorganic material biphasic microspheres are first prepared, physical electrostatic crosslinking or chemical crosslinking is then performed on carboxyl groups at Polylactic Acid terminals in the biphasic microspheres and the hyaluronic acid, and the formed triphasic composite gel is high in stability and good in biocompatibility, and may be used for filling and repairing of biological tissue.

(2) For the biphasic microspheres in the present disclosure, due to the addition of a hydrophilic calcium salt inorganic material (such as Hydroxyapatite, calcium phosphate, calcium tartrate, etc.), the hydrophilicity of the Polylactic Acid microspheres is further improved. In addition, a calcium-based inorganic material in the biphasic microspheres may use micron-sized Polylactic Acid as a carrier, and is introduced into a network structure of the macroscopic cross-linked hyaluronic acid (cross-linked hyaluronic acid particles are also micron-sized); through the complexing action of calcium ions with carboxyl and hydroxyl groups in the hyaluronic acid, network gaps among the cross-linked hyaluronic acid are filled up, such that the triphasic gel has a tighter network structure; and meanwhile, in combination with the complexing action of the calcium-based inorganic material with the hyaluronic acid, a distance between the Polylactic Acid and a hyaluronic acid molecular chain is shortened, such that the efficiency of the crosslinking reaction between them is improved, and the mechanical strength and thermal stability of the triphasic composite gel are improved.

(3) The preparation method of the present disclosure is simple in process, convenient in operation, and each to realize industrialization.

(4) The composite gel prepared in the present disclosure may realize an immediate filling effect of the hyaluronic acid, a collagen regeneration effect of the Polylactic Acid, and the effect of the calcium-based inorganic material such as the Hydroxyapatite for bone filling and neutralization of lactic acid, integrating the advantages of the three materials.

## Brief Description of the Drawings

In order to more clearly illustrate the specific implementations of the present disclosure or the technical solutions in the related art, the drawings used in the description of the specific implementations or the related art will be briefly described below. It is apparent that the drawings in the following descriptions are some implementations of the present disclosure. Other drawings can be obtained from those skilled in the art according to these drawings without any creative work.

Fig. 1 is a microscope photograph of Hydroxyapatite dispersed Polylactic Acid biphasic microspheres according to Embodiment 1 of the present disclosure.

Fig. 2 is a scanning electron micrograph of Hydroxyapatite dispersed Polylactic Acid biphasic microspheres according to Embodiment 1 of the present disclosure.

Fig. 3 is a partial enlarged scanning electron micrograph of Hydroxyapatite dispersed Polylactic Acid biphasic microspheres according to Embodiment 1 of the present disclosure.

## Detailed Description of the Embodiments

The technical solutions of the present disclosure will be clearly and completely described below with reference to the embodiments. It is apparent that the described embodiments are merely part of the embodiments of the present disclosure, not all the embodiments. Based on the embodiments in the present disclosure, all other embodiments obtained by those of ordinary skill in the art without creative work shall fall within the protection scope of the present disclosure.

Embodiment 1: Biphasic microsphere preparation 1

1.8 g of Polylactic Acid was weighed and placed in a 50 mL beaker, then 50 mL of dichloromethane was added and stirred with a spoon to make it completely dissolved, then the mixture was filtered into a ultrasound dedicated beaker by using a 10 mm nylon 66 syringe filter, then 0.2 g of nano Hydroxyapatite was weighed and added to the system, the system was acted for 10 min with 60% power (12 KJ) to fully disperse the nano material into the dichloromethane solution of the Polylactic Acid, in the meantime, 135 mL of a polyvinyl alcohol solution (1.5 wt%) was added to a 300 mL three-necked flask and placed in a 30°C water bath, and then mechanical stirring was performed at a rate of 400 rpm. After ultrasound ended, a dichloromethane turbid solution was slowly added to the polyvinyl alcohol solution, and stirring was performed overnight to remove the dichloromethane through volatilization.

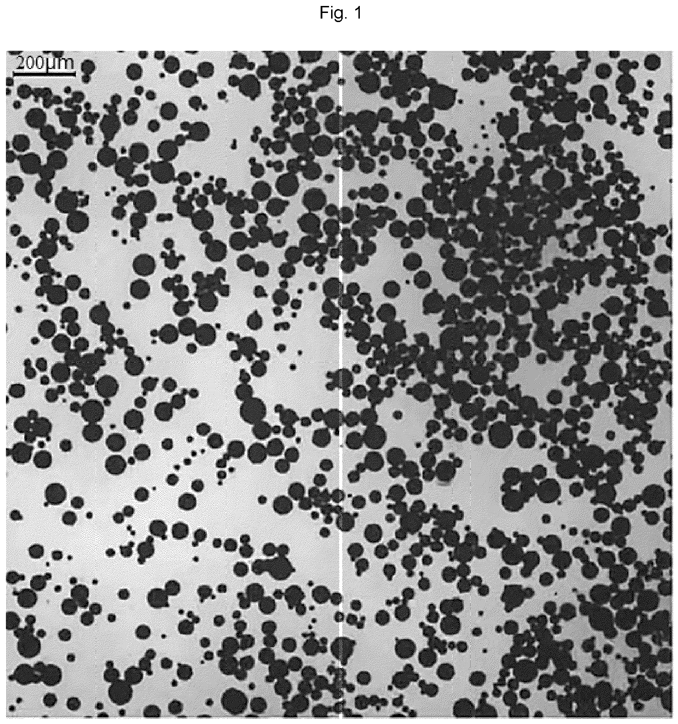

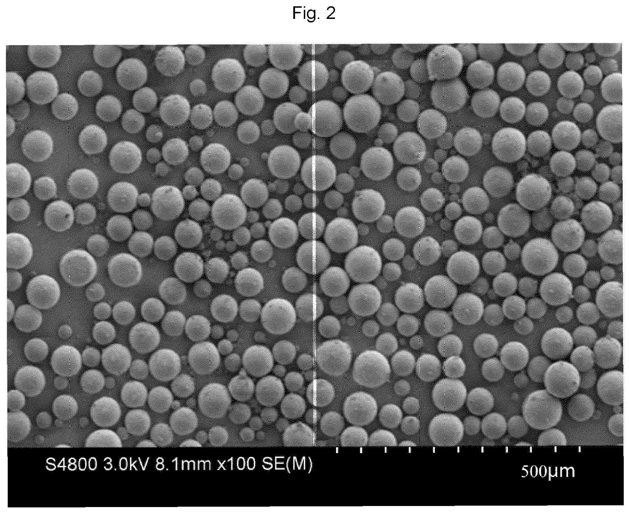

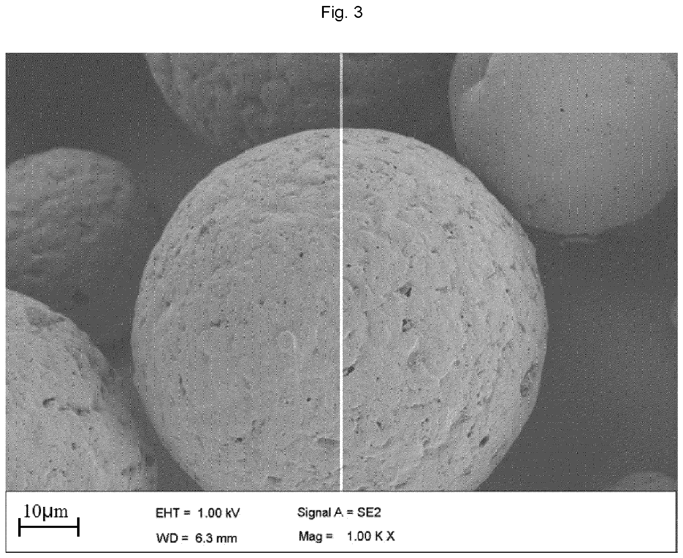

The turbid solution obtained from the previous step was added with purified water to dilute to 500 mL, and then was allowed to stand for 1 h; upper suspension was poured; the operation was repeated for two times; then precipitation was re-dissolved; filtration was performed by using a 0.45 μm water system filter membrane, and liquid was removed; then a solid obtained was dispersed with 200 mL purified water; filtration was performed again; the operation was repeated for four times; then a solid obtained was re-dissolved with 150 mL purified water, and added to a Schott bottle; the Schott bottle was placed to a 100°C oven to heat for 30 min, then taken out, and filtered after cooling; washing was performed with purified water for three times, and a solid obtained from the last wash was transferred to a culture dish; and the culture dish was placed in a vacuum oven to dry overnight at 40°C, so as to obtain a final product. The particle size of the product was mainly distributed between 25 and 86 μm, a microscope photograph was shown in Fig. 1, and a scanning electron micrograph was shown in Figs. 2-3. From the figures, it might be seen that the biphasic microspheres are regular in morphology, and uniform in particle size.

Embodiment 2: Biphasic microsphere preparation 2

1.6 g of Polylactic Acid was weighed and placed in a 50 mL beaker, then 50 mL of dichloromethane was added and stirred with a spoon to make it completely dissolved, then the mixture was filtered into a ultrasound dedicated beaker by using a 10 mm nylon 66 syringe filter, then 0.2 g of nano Hydroxyapatite was weighed and added to the system, the system was acted for 10 min with 60% power (12 KJ) to fully disperse the nano material into the dichloromethane solution of the Polylactic Acid, in the meantime, 90 mL of a polyvinyl alcohol solution (1.5 wt%) was added to a 300 mL three-necked flask and placed in a 30°C water bath, and then mechanical stirring was performed at a rate of 400 rpm. After ultrasound ended, a dichloromethane turbid solution was slowly added to the polyvinyl alcohol solution, and stirring was performed overnight to remove the dichloromethane through volatilization.

The turbid solution obtained from the previous step was added with purified water to dilute to 500 mL, and then was allowed to stand for 1 h; upper suspension was poured; the operation was repeated for two times; then precipitation was re-dissolved; filtration was performed by using a 0.45 μm water system filter membrane, and liquid was removed; then a solid obtained was dispersed with 200 mL purified water; filtration was performed again; the operation was repeated for four times; then a solid obtained was re-dissolved with 150 mL purified water, and added to a Schott bottle; the Schott bottle was placed to a 100°C oven to heat for 30 min, then taken out, and filtered after cooling; washing was performed with purified water for three times, and a solid obtained from the last wash was transferred to a culture dish; and freeze drying was performed to obtain a final product. A particle size was mainly distributed between 25 and 72 μm.

Embodiment 3: Physical crosslinking of a triphasic gel

1.5 g of sodium hyaluronate (molecular weight 900 KDa) was weighed, then 37.5 mL of purified water was added, after complete dissolving, the current concentration of hyaluronic acid was 40 mg/mL, and 45 mg of spermidine was added to the hyaluronic acid solution. In the presence of three-site crosslinking of the spermidine, a pH value of the hyaluronic acid solution was regulated to about 6.2 by using a 6 mol/L hydrochloric acid solution, then uniformly stirred, 0.214 g of EDC was added, 0.04 g of NHS was simultaneously added and continuously uniformly stirred, and the mixture was sealed and placed in a 40°C drying box for reaction for 14 h. After the reaction ended, 40 mL of anhydrous ethanol was added, gel particles were smashed for 5 min by using an IKA T25 high shear disperser at a smashing rotary speed of 10,000 rpm. After smashing ended, 200 mL of the anhydrous ethanol was continuously added to make the gel completely dehydrated. Precipitation was separated, the precipitation was continuously washed for 5 times by using 200 mL of the anhydrous ethanol, then the precipitation was placed in a vacuum oven, and vacuum drying was performed for 24 h at 40°C and a vacuum degree of -0.09 MPa. After complete drying, 1.0 g of a dry gel was taken, 50 mL of PBS with pH 6.0 and concentration being 10mg/mL was added in total, and the gel was completely swelled for later use.

3.0 g of the biphasic microspheres obtained in Embodiment 1 were taken, and soaked in 50 mL of a PBS with pH 9.0 for surface charge modification, to make the surfaces of the microspheres carry negative charges; after 30 minutes of soaking, the biphasic microspheres were separated, taken out, dried, and physically mixed with 50 mL of cross-linked hyaluronic acid gel, so as to cause negative carboxylate ions on the surface of the Polylactic Acid to be subjected to electrostatic crosslinking with positive ammonium ions in the cross-linked hyaluronic acid. After well mixing, the triphasic gel was filled in a prefilled syringe, and moist heat sterilization was performed for 15 min at 121°C, so as to obtain a final product gel.

Embodiment 4: Chemical crosslinking of a triphasic gel

1.5 g of the biphasic microspheres obtained in Embodiment 1 were taken, 100 mL of purified water was added, 2.0 g of ethylenediamine was added, the pH of the solution was regulated to 5.5 by using a 1mol/L hydrochloric acid solution, 2.0 g of EDC and 0.4 g of NHS were added to perform a surface amino modification reaction, and the reaction was performed for 16 h at 40°C. The microspheres were filtered and separated, the microspheres were washed with purified water until pH was within pH 7.0-7.1, and drying was performed overnight at 40°C in a vacuum oven, so as to obtain the biphasic microspheres of which surfaces were modified with amino.

1.5 g of sodium hyaluronate was weighed, then 37.5 mL of purified water was added, after complete dissolving, the current concentration of hyaluronic acid was 40 mg/mL, and 1.0 g of the biphasic microspheres with amino modified surface were added to the hyaluronic acid solution. A pH value of the hyaluronic acid solution was regulated to about 5.5 by using a 0.1 mol/L hydrochloric acid solution, then uniformly stirred, 0.3 g of EDC was added, 0.06 g (20% of the mass of the EDC) of NHS was simultaneously added and continuously uniformly stirred, and the mixture was sealed and placed in a 40°C drying box for reaction for 14 h. After the reaction ended, 200 mL of anhydrous ethanol was added to make the gel completely dehydrated. Precipitation was separated, the precipitation was continuously washed for 5 times by using 200 mL of the anhydrous ethanol, then the precipitation was placed in a vacuum oven, and vacuum drying was performed for 24 h at 40°C and a vacuum degree of -0.09 MPa. After complete drying, 1.0 g of a dry gel was taken, and 50 mL of a phosphate buffer solution with pH being 7.0 and concentration being 10 mg/mL was added in total; and after the gel was completely swelled, the gel was filled in a prefilled syringe, and moist heat sterilization was performed for 15 min at 121°C, so as to obtain a final product triphasic gel.

Embodiment 5: Preparation of calcium carbonate biphasic microspheres

1.6 g of Polylactic Acid was weighed and placed in a 50 mL beaker, then 50 mL of dichloromethane was added and stirred with a spoon to make it completely dissolved, then the mixture was filtered into a ultrasound dedicated beaker by using a 10mm nylon 66 syringe filter, then 0.2 g of nano calcium carbonate was weighed and added to the system, the system was acted for 10 min with 60% power (12 KJ) to fully disperse the nano material into the dichloromethane solution of the Polylactic Acid, in the meantime, 90mL of a polyvinyl alcohol solution (1.5 wt%) was added to a 300 mL three-necked flask and placed in a 30°C water bath, and then mechanical stirring was performed at a rate of 400 rpm. After ultrasound ended, a dichloromethane turbid solution was slowly added to the polyvinyl alcohol solution, and stirring was performed overnight to remove the dichloromethane through volatilization.
The turbid solution obtained from the previous step was added with purified water to dilute to 500 mL, and then was allowed to stand for 1 h; upper suspension was poured; the operation was repeated for two times; then precipitation was re-dissolved; filtration was performed by using a 0.45 μm water system filter membrane, and liquid was removed; then a solid obtained was dispersed with 200 mL purified water; filtration was performed again; the operation was repeated for four times; then a solid obtained was re-dissolved with 150 mL purified water, and added to a Schott bottle; the Schott bottle was placed to a 100°C oven to heat for 30 min, then taken out, and filtered after cooling; washing was performed with purified water for three times, and a solid obtained from the last wash was transferred to a culture dish; and freeze drying was performed to obtain a final product.

Embodiment 6: Preparation of calcium tartrate biphasic microspheres

1.6 g of Polylactic Acid was weighed and placed in a 50 mL beaker, then 50 mL of dichloromethane was added and stirred with a spoon to make it completely dissolved, then the mixture was filtered into a ultrasound dedicated beaker by using a 10mm nylon 66 syringe filter, then 0.2 g of nano calcium tartrate was weighed and added to the system, the system was acted for 10 min with 60% power (12 KJ) to fully disperse the nano material into the dichloromethane solution of the Polylactic Acid, in the meantime, 90 mL of a polyvinyl alcohol solution (1.5 wt%) was added to a 300mL three-necked flask and placed in a 30°C water bath, and then mechanical stirring was performed at a rate of 400 rpm. After ultrasound ended, a dichloromethane turbid solution was slowly added to the polyvinyl alcohol solution, and stirring was performed overnight to remove the dichloromethane through volatilization.
The turbid solution obtained from the previous step was added with purified water to dilute to 500 mL, and then was allowed to stand for 1 h; upper suspension was poured; the operation was repeated for two times; then precipitation was re-dissolved; filtration was performed by using a 0.45 μm water system filter membrane, and liquid was removed; then a solid obtained was dispersed with 200 mL purified water; filtration was performed again; the operation was repeated for four times; then a solid obtained was re-dissolved with 150 mL purified water, and added to a Schott bottle; the Schott bottle was placed to a 100°C oven to heat for 30 min, then taken out, and filtered after cooling; washing was performed with purified water for three times, and a solid obtained from the last wash was transferred to a culture dish; and freeze drying was performed to obtain a final product.

Embodiment 7: Chemical crosslinking of a calcium carbonate triphasic gel

1.5 g of the biphasic microspheres obtained in Embodiment 5 were taken, 100 mL of purified water was added, 2.0 g of ethylenediamine was added, the pH of the solution was regulated to 5.5 by using a 1 mol/L hydrochloric acid solution, 2.0 g of EDC and 0.4 g of NHS were added to perform a surface amino modification reaction, and the reaction was performed for 16 h at 40°C. The microspheres were filtered and separated, the microspheres were washed with purified water until pH was within pH 7.0-7.1, and drying was performed overnight at 40°C in a vacuum oven, so as to obtain the biphasic microspheres of which surfaces were modified with amino.
1.5g of sodium hyaluronate was weighed, and then 37.5 mL of purified water was added, after complete dissolving, the current concentration of hyaluronic acid was 40 mg/mL, and 1.0 g of biphasic microspheres with amino modified surface were added to the hyaluronic acid solution. A pH value of the hyaluronic acid solution was regulated to about 5.5 by using a 0.1 mol/L hydrochloric acid solution, then the hyaluronic acid solution was added and uniformly stirred, 0.3 g of EDC was added, 0.06 g (20% of the mass of the EDC) of NHS was simultaneously added and continuously uniformly stirred, and the mixture was sealed and placed in a 40°C drying box for reaction for 14 h. After the reaction ended, 200 mL of anhydrous ethanol was added to make the gel completely dehydrated. Precipitation was separated, the precipitation was continuously washed for 5 times by using 200 mL of the anhydrous ethanol, then the precipitation was placed in a vacuum oven, and vacuum drying was performed for 24 h at 40°C and a vacuum degree of -0.09 MPa. After complete drying, 1.0 g of a dry gel was taken, and 50 mL of a phosphate buffer solution with pH being 7.0 and concentration being 10 mg/mL was added in total; and after the gel was completely swelled, the gel was filled in a prefilled syringe, and moist heat sterilization was performed for 15 min at 121°C, so as to obtain a final product triphasic gel.

Embodiment 8: Chemical crosslinking of a calcium tartrate triphasic gel

1.5 g of the biphasic microspheres obtained in Embodiment 6 were taken, 100 mL of purified water was added, 2.0 g of ethylenediamine was added, the pH of the solution was regulated to 5.5 by using a 1 mol/L hydrochloric acid solution, 2.0 g of EDC and 0.4 g of NHS were added to perform a surface amino modification reaction, and the reaction was performed for 16 h at 40°C. The microspheres were filtered and separated, the microspheres were washed with purified water until pH was within pH 7.0-7.1, and drying was performed overnight at 40°C in a vacuum oven, so as to obtain the biphasic microspheres of which surfaces were modified with amino.
1.5 g of sodium hyaluronate was weighed, and then 37.5 mL of purified water was added, after complete dissolving, the current concentration of hyaluronic acid was 40 mg/mL, and 1.0 g of biphasic microspheres with amino modified surface were added to the hyaluronic acid solution. A pH value of the hyaluronic acid solution was regulated to about 5.5 by using a 0.1mol/L hydrochloric acid solution, then the hyaluronic acid solution was added and uniformly stirred, 0.3 g of EDC was added, 0.06 g (20% of the mass of the EDC) of NHS was simultaneously added and continuously uniformly stirred, and the mixture was sealed and placed in a 40°C drying box for reaction for 14 h. After the reaction ended, 200 mL of anhydrous ethanol was added to make the gel completely dehydrated. Precipitation was separated, the precipitation was continuously washed for 5 times by using 200 mL of the anhydrous ethanol, then the precipitation was placed in a vacuum oven, and vacuum drying was performed for 24 h at 40°C and a vacuum degree of -0.09 MPa. After complete drying, 1.0 g of a dry gel was taken, and 50 mL of a phosphate buffer solution with pH being 7.0 and concentration being 10mg/mL was added in total; and after the gel was completely swelled, the gel was filled in a prefilled syringe, and moist heat sterilization was performed for 15 min at 121°C, so as to obtain a final product triphasic gel.

Comparative embodiment 1 Preparation of a non-cross-linked triphasic gel

1.5 g of sodium hyaluronate was weighed, and then 37.5 mL of purified water was added, after complete dissolving, the current concentration of hyaluronic acid was 40 mg/mL, and 1.0 g of the biphasic microspheres obtained in Embodiment 2 were added to the hyaluronic acid solution and uniformly stirred. After stirring ended, 200 mL of anhydrous ethanol was added to make the gel completely dehydrated. Precipitation was separated, the precipitation was continuously washed for 5 times by using 200 mL of the anhydrous ethanol, then the precipitation was placed in a vacuum oven, and vacuum drying was performed for 24 h at 40°C and a vacuum degree of -0.09 MPa. After complete drying, 1.0 g of a dry gel was taken, and 50 mL of a phosphate buffer solution with pH being 7.0 and concentration being 10 mg/mL was added in total; and after the gel was completely swelled, the gel was filled in a prefilled syringe, and moist heat sterilization was performed for 15 min at 121°C, so as to obtain a final product non-cross-linked triphasic gel.

Comparative embodiment 2 Preparation of a triphasic non-physical crosslinking blended gel

A method for preparing a spermidine-cross-linked hyaluronic acid gel was the same as Embodiment 3, and the overall preparation method was identical.
3.0 g of the biphasic microspheres obtained in Embodiment 1 were taken, and directly physically mixed with 50 mL of a cross-linked hyaluronic acid gel. After well mixing, the triphasic gel was filled in a prefilled syringe, and moist heat sterilization was performed for 15 min at 121°C, so as to obtain a final product gel.

Comparative embodiment 3 Preparation of a monophasic PLA microsphere and cross-linked hyaluronic acid gel physically-blended gel

1.6 g of Polylactic Acid was weighed and placed in a 50 mL beaker, and then 50 mL of dichloromethane was added and stirred with a spoon to make it completely dissolved, then the mixture was filtered into an ultrasound dedicated beaker by using a 10 mm nylon 66 syringe filter, in the meantime, 90 mL of a polyvinyl alcohol solution (1.5wt%) was added to a 300 mL three-necked flask and placed in a 30°C water bath, and then mechanical stirring was performed at a rate of 400 rpm. A dichloromethane solution was slowly added to the polyvinyl alcohol solution, and stirring was performed overnight to remove the dichloromethane through volatilization. The turbid solution obtained from the previous step was added with purified water to dilute to 500 mL, and then was allowed to stand for 1 h; upper suspension was poured; the operation was repeated for two times; then precipitation was re-dissolved; filtration was performed by using a 0.45 $\mu$m water system filter membrane, and liquid was removed; then a solid obtained was dispersed with 200 mL purified water; filtration was performed again; the operation was repeated for four times; then a solid obtained was re-dissolved with 150 mL purified water, and added to a Schott bottle; the Schott bottle was placed to a 100°C oven to heat for 30 min, then taken out, and filtered after cooling; washing was performed with purified water for three times, and a solid obtained from the last wash was transferred to a culture dish; and freeze drying was performed to obtain a final product-PLA microspheres.

3.0 g of the obtained PLA microspheres were taken, and soaked in 50 mL of a PBS with pH being 9.0 for surface charge modification, to make the surfaces of the microspheres carry negative charges; after 30 minutes of soaking, the microspheres were separated, taken out, dried, and physically mixed with 50 mL of cross-linked hyaluronic acid gel obtained in Embodiment 3, so as to cause negative carboxylate ions on the surface of the PLA to be subjected to electrostatic crosslinking with positive ammonium ions in the cross-linked hyaluronic acid. After well mixing, the blended gel was filled in a prefilled syringe, and moist heat sterilization was performed for 15 min at 121°C, so as to obtain a final product gel.

Comparative embodiment 4 Preparation of a nanoscale HAP and cross-linked hyaluronic acid gel physically-blended gel.

A method for preparing a spermidine-cross-linked hyaluronic acid gel was the same as Embodiment 3, and the overall preparation method was identical.

3.0 g of the nanoscale HAP was taken, and directly physically mixed with 50 mL of a cross-linked hyaluronic acid gel. After well mixing, the blended gel was filled in a prefilled syringe, and moist heat sterilization was performed for 15 min at 121°C, so as to obtain a final product gel.

Effect embodiment:

Detection of rheological property of composite gels

In the composite gels obtained in the embodiments and comparative embodiments were divided into two types: before and after sterilization, 2.0 mL of each was taken, the elastic modulus (G') of the gel was detected by using a rheometer with parallel plates, and a loss ratio of the elastic modulus was calculated. The G' loss ratio was calculated by the following formula.

$$\text{G' loss ratio = (G' before sterilization - G' after sterilization)/G' before sterilization}$$

Parameters of the rheometer included: operation gap: 1000 mm; loading gap: 45000 m; operating temperature: 37 °C; deformation quantity: 1 %; frequency: 0.9 Hz; and operating time: 60 s. Gel rheology data was shown in Table 1.

Table 1 Rheological data of composite gels

| Embodiment/Comparative embodiment | G' before sterilization (Pa) | G' after sterilization (Pa) | G' loss ratio |
|---|---|---|---|
| Embodiment 3 | 473 | 344 | 27.3% |
| Embodiment 4 | 867 | 698 | 19.5% |
| Embodiment 7 | 813 | 615 | 24.4% |
| Embodiment 8 | 837 | 618 | 26.2% |
| Comparative embodiment 1 | 372 | 10 | 97.3% |
| Comparative embodiment 2 | 403 | 256 | 36.5% |
| Comparative embodiment 3 | 275 | 166 | 39.6% |
| Comparative embodiment 4 | 266 | 153 | 42.5% |

From the data of Table 1, it might be seen that, compared to the triphasic non-physical crosslinking blended gel of Comparative embodiment 2, the elastic modulus of the triphasic gel obtained through physical crosslinking in Embodiment 3 was increased, and thermal stability was improved to a certain extent, indicating that physical crosslinking contributed to the increasing of the elastic modulus and the thermal stability of the composite gel. Since the non-cross-linked HA, the PLA, and the HAP were only physically mixed in Comparative embodiment 1, and there was no chemical bonding in the three substances, the three substances were unable to resist intense hydrolysis during moist heat sterilization, causing a decreased value of the elastic modulus of Comparative embodiment 1 after sterilization to exceed 97%.

In Comparative embodiment 3, although the PLA and the HA were physically crosslinked, the HAP was not added, the elastic modulus of the composite gel was lower than that in Embodiment 3, and the G' loss ratio due to heat was high. It indicated that, after the HAP formed the composite microspheres with the PLA, the nano HAP used micron-sized PLA as a carrier, and was introduced into a network structure of the macroscopic cross-linked hyaluronic acid (cross-linked hyaluronic acid particles are also micron-sized); through the complexing action of calcium ions with carboxyl and hydroxyl groups in the hyaluronic acid, network gaps among the cross-linked hyaluronic acid are filled up, such that the triphasic gel has a tighter network structure; and meanwhile, in combination with the complexing action of the HAP and the HA, a distance between the PLA and a HA molecular chain was shortened, such that the efficiency of the crosslinking reaction between them was improved, and the composite gel had higher elastic modulus and stronger thermal stability.

Comparative embodiment 4 disclosed the physical blending of the nanoscale HAP and the cross-linked hyaluronic acid gel, but did not show high elastic modulus and strong thermal stability. The reason was that, Comparative embodiment 4, the nanoscale HAP was directly added, compared with the micron-sized cross-linked hyaluronic acid gel, there was a large difference in physical sizes of the two substances, resulting in a large space difference between them, such that the HAP is difficult to fill gaps of the cross-linked hyaluronic acid gel, so as to be unable to form a tighter network structure. This further indicated that, by using the HAP and the PLA to form the biphasic microsphere, the HAP was carried on the micron-sized carrier of the PLA, which might further form a tighter network structure with the cross-linked hyaluronic acid gel, so as to achieve higher elastic modulus and stronger thermal stability. Therefore, the composite gel was based on the synergistic interaction between the cross-linked hyaluronic acid and PLA-HAP biphasic microspheres, and the elastic modulus and thermal stability in the triphasic gel were improved, and the three components in the triphasic gel were indispensable.

The elastic modulus of the triphasic composite gel obtained through chemical crosslinking in Embodiments 4, 7, and 8 was significantly increased, indicating that, when physical crosslinking was replaced with chemical crosslinking, since the strength of bonding interactions formed by chemical crosslinking was much higher than the interaction strength of physical crosslinking, the interaction among the cross-linked hyaluronic acid, the HAP (or other nanoscale calcium salts), and the PLA was further strengthened. Compared to the triphasic gel of physical crosslinking, the elastic modulus and thermal stability of the triphasic gel of chemical crosslinking were further improved.

## Claims

1. A composite gel, prepared by crosslinking biphasic microspheres with hyaluronic acid, wherein the biphasic microspheres are Polylactic Acid/inorganic material biphasic microspheres;

   the crosslinking comprises crosslinking of Polylactic Acid in the biphasic microspheres with the hyaluronic acid;
   the Polylactic Acid is micron-sized;
   the inorganic material is selected from one or more of calcium carbonate, calcium phosphate, and calcium tartrate;
   the inorganic material is a nano inorganic material.

2. The composite gel according to claim 1, wherein the crosslinking of the Polylactic Acid with the hyaluronic acid comprises any one of physical electrostatic crosslinking and chemical crosslinking.

3. The composite gel according to claim 1 or 2, wherein the calcium phosphate is Hydroxyapatite.

4. The composite gel according to any one of claims 1 to 3, wherein the hyaluronic acid is cross-linked hyaluronic acid; further, the cross-linked hyaluronic acid is prepared through a crosslinking agent, and the crosslinking agent is selected from one or more of dialdehyde, disulfide, Polyethylene Glycol (PEG) crosslinking agent, divinyl sulphone, diglycidyl ether, di-epoxide, and polyamine; and further, the crosslinking agent is endogenous polyamine.

5. The composite gel according to any one of claims 1-4, wherein the composite gel is prepared by crosslinking hyaluronic acid with Polylactic Acid biphasic microspheres in which nanoscale Hydroxyapatite is dispersed wherein the mass of the Hydroxyapatite accounts for 0.1-50% of the total mass of the Hydroxyapatite dispersed Polylactic Acid biphasic microspheres.

6. A method for preparing a composite gel according to any one of claims 1-5, comprising crosslinking biphasic microspheres with hyaluronic acid to form a composite gel, wherein the biphasic microspheres are Polylactic Acid/inorganic material biphasic microspheres, and the crosslinking comprises crosslinking of Polylactic Acid in the biphasic microspheres with the hyaluronic acid.

7. The preparation method according to claim 6, wherein the crosslinking is solid phase crosslinking; and further, the solid phase crosslinking comprises any one of physical electrostatic crosslinking and chemical crosslinking.

8. The preparation method according to claim 6 or 7, wherein the hyaluronic acid is cross-linked hyaluronic acid; the cross-linked hyaluronic acid is prepared through a crosslinking agent; and the crosslinking agent is selected from one or more of dialdehyde, disulfide, a Polyethylene Glycol (PEG) crosslinking agent, divinyl sulphone, diglycidyl ether, di-epoxide and polyamine.

9. The preparation method according to any one of claims 6-8, wherein the chemical crosslinking comprises performing amino modification on the Polylactic Acid in the biphasic microspheres, and then reacting the modified biphasic microspheres with the hyaluronic acid to form amide bond crosslinking; further, the amino modification comprises reacting the biphasic microspheres with an amine compound; and further the amide bond crosslinking comprises performing a coupling reaction on the modified biphasic microspheres and the hyaluronic acid under the presence of a carbodiimide activating agent.

10. The preparation method according to claim 9, wherein the amine compound contains polyamine or a polyamino compound;

   preferably, the polyamine comprises any one of aliphatic polyamine, aromatic polyamine, and heteroatom polyamine; the polyamino compound comprises any one of bis-terminal aminated PEG and terminal aminated multiarm PEG; and
   further, an addition amount of the amine compound is 30-200% of the mass of the biphasic microspheres, and the concentration of the amine compound ranges from 10 to 1000 mg/mL.

11. The preparation method according to any one of claims 7-10, wherein a mass ratio of the biphasic microspheres to the hyaluronic acid in the chemical crosslinking is 1:0.5-20, and is preferably 1:1-10;

   further, the carbodiimide activating agent comprises one or more of 1-ethyl-3-(3-dimethylaminopropyl)carbodiimide, 1-(3-dimethylaminopropyl)-3-ethyl-carbodiimide, 1-cyclohexyl-3-(2-morpholinoethyl)carbodiimide, 1,3-bis[di(methoxymethyl)methyl]carbodiimide, and salts thereof;
   further, an addition amount of the carbodiimide activating agent is 10-150% of the total mass of the hyaluronic acid;
   further, the concentration of a hyaluronic acid solution is 10-100 mg/mL; and
   further, reaction temperatures of the amino modification and the amide bond crosslinking are 5-60°C, and reaction time is 6-24 h.

12. The preparation method according to claim 7 or 8, wherein the physical electrostatic crosslinking comprises performing surface charge modification on the biphasic microspheres to obtain modified biphasic microspheres with surfaces carrying negative ions, and mixing the modified biphasic microspheres and cross-linked hyaluronic acid having positive ions, so as to perform electrostatic crosslinking; further, the cross-linked hyaluronic acid having positive ions is cross-linked hyaluronic acid obtained through polyamine crosslinking; and further, the mass of the polyamine crosslinking agent accounts for 0.5-20% of the total mass of the hyaluronic acid;
   and further, performing surface charge modification on the biphasic microspheres comprises soaking the biphasic microspheres in an alkaline buffer solution, and obtaining the modified biphasic microspheres through separation.

13. The preparation method according to claim 7, 8 or 12, wherein a mass ratio of the biphasic microspheres to the cross-linked hyaluronic acid in the physical electrostatic crosslinking is 1:1-50, and is preferably 1:1-20; and
   further, the alkaline buffer solution is an alkaline phosphate buffer solution, and the pH of the alkaline phosphate buffer solution preferably ranges from 9.0 to 11.0.

14. An application of the composite gel according to any one of claims 1 to 5 or the composite gel prepared by the preparation method according to any one of claims 6 to 13 in preparation of drug carriers, fillers, or tissue repairing materials.

**Patentansprüche**

1. Verbundgel, hergestellt durch Vernetzen von zweiphasigen Mikrokügelchen mit Hyaluronsäure, wobei die zweiphasigen Mikrokügelchen Polymilchsäure-/anorganisches-Material- zweiphasige Mikrokügelchen sind,

   wobei das Vernetzen ein Vernetzen von Polymilchsäure in den zweiphasigen Mikrokügelchen mit der Hyaluronsäure aufweist,
   die Polymilchsäure mikrometergroß ist,
   das anorganische Material aus einem oder mehreren von Calciumcarbonat, Calciumphosphat und Calciumtartrat ausgewählt ist,
   das anorganische Material ein nanoanorganisches Material ist.

2. Verbundgel gemäß Anspruch 1, wobei das Vernetzen der Polymilchsäure mit der Hyaluronsäure irgendeines von einem physikalischen elektrostatischen Vernetzen und einem chemischen Vernetzen aufweist.

3. Verbundgel gemäß Anspruch 1 oder 2, wobei das Calciumphosphat Hydroxylapatit ist.

4. Verbundgel gemäß irgendeinem der Ansprüche 1 bis 3, wobei die Hyaluronsäure vernetzte Hyaluronsäure ist, ferner die vernetzte Hyaluronsäure durch ein Vernetzungsmittel hergestellt wird und das Vernetzungsmittel aus einem oder mehreren von Dialdehyd, Disulfid, Polyethylenglykol- (PEG)-Vernetzungsmittel, Divinylsulfon, Diglycidylether, Diepoxid und Polyamin ausgewählt ist, und ferner das Vernetzungsmittel endogenes Polyamin ist.

5. Verbundgel gemäß irgendeinem der Ansprüche 1 bis 4, wobei das Verbundgel durch Vernetzen von Hyaluronsäure mit Polymilchsäure- zweiphasigen Mikrokügelchen hergestellt ist, in denen nanoskaliges Hydroxylapatit dispergiert ist, wobei die Masse des Hydroxylapatits 0,1 bis 50 % der Gesamtmasse der Hydroxylapatit-dispergierten Polymilchsäure- zweiphasigen Mikrokügelchen ausmacht.

6. Verfahren zum Herstellen eines Verbundgels gemäß irgendeinem der Ansprüche 1-5, aufweisend das Vernetzen von zweiphasigen Mikrokügelchen mit Hyaluronsäure, um ein Verbundgel zu bilden, wobei die zweiphasigen Mikrokügelchen Polymilchsäure-/anorganisches-Material- zweiphasige Mikrokügelchen sind und das Vernetzen ein Vernetzen von Polymilchsäure in den zweiphasigen Mikrokügelchen mit der Hyaluronsäure aufweist.

7. Herstellungsverfahren gemäß Anspruch 6, wobei das Vernetzen ein Festphasenvernetzen ist und ferner das Festphasenvernetzen irgendeines von einem physikalischen elektrostatischen Vernetzen und einem chemischen Vernetzen aufweist.

8. Herstellungsverfahren gemäß Anspruch 6 oder 7, wobei die Hyaluronsäure vernetzte Hyaluronsäure ist, die vernetzte Hyaluronsäure durch ein Vernetzungsmittel hergestellt wird, und das Vernetzungsmittel aus einem oder mehreren von Dialdehyd, Disulfid, einem Polyethylenglykol- (PEG)-Vernetzungsmittel, Divinylsulfon, Diglycidylether, Diepoxid und Polyamin ausgewählt ist.

9. Herstellungsverfahren gemäß irgendeinem der Ansprüche 6 bis 8, wobei das chemische Vernetzen das Durchführen einer Aminomodifizierung an der Polymilchsäure in den zweiphasigen Mikrokügelchen und anschließendes Reagieren der modifizierten zweiphasigen Mikrokügelchen mit der Hyaluronsäure zum Ausbilden einer Amidbindungsvernetzung aufweist, wobei die Aminomodifizierung ferner das Reagieren der zweiphasigen Mikrokügelchen mit einer Aminverbindung aufweist, und ferner die Amidbindungsvernetzung das Durchführen einer Kupplungsreaktion an den modifizierten zweiphasigen Mikrokügelchen und der Hyaluronsäure in Gegenwart eines Carbodiimid-Aktivierungsmittels aufweist.

10. Herstellungsverfahren gemäß Anspruch 9, wobei die Aminverbindung Polyamin oder eine Polyaminverbindung aufweist,

    wobei das Polyamin vorzugsweise irgendeines von aliphatischem Polyamin, aromatischem Polyamin und Heteroatom-Polyamin aufweist, die Polyaminverbindung irgendeines von bis-terminal-aminiertem PEG und terminal-aminiertem mehrarmigem PEG aufweist, und
    ferner eine Zugabemenge der Aminverbindung 30 bis 200 % der Masse der zweiphasigen Mikrokügelchen beträgt und die Konzentration der Aminverbindung von 10 bis 1000 mg/ml beträgt.

11. Herstellungsverfahren gemäß irgendeinem der Ansprüche 7 bis 10, wobei ein Massenverhältnis der zweiphasigen Mikrokügelchen zu der Hyaluronsäure bei der chemischen Vernetzung 1:0,5-20 beträgt und vorzugsweise 1:1-10 beträgt,

ferner das Carbodiimid-Aktivierungsmittel eines oder mehrere von 1-Ethyl-3-(3-dimethylaminopropyl)carbodiimid, 1-(3-Dimethylaminopropyl)-3-ethylcarbodiimid, 1-Cyclohexyl-3-(2-morpholinoethyl)carbodiimid, 1,3-Bis [di(methoxymethyl)methyl]carbodiimid und Salze davon aufweist,
ferner eine Zugabemenge des Carbodiimid-Aktivierungsmittels 10-150 % der Gesamtmasse der Hyaluronsäure beträgt,
ferner die Konzentration einer Hyaluronsäurelösung 10 bis 100 mg/ml beträgt, und
ferner Reaktionstemperaturen der Aminomodifizierung und der Amidbindungsvernetzung 5 bis 60 °C betragen und eine Reaktionszeit 6 bis 24 h beträgt.

12. Herstellungsverfahren gemäß Anspruch 7 oder 8, wobei das physikalische elektrostatische Vernetzen aufweist: Durchführen einer Oberflächenladungsmodifizierung an den zweiphasigen Mikrokügelchen, um modifizierte zweiphasige Mikrokügelchen mit Flächen zu erhalten, die negative Ionen tragen, und Mischen der modifizierten zweiphasigen Mikrokügelchen und der vernetzten Hyaluronsäure mit positiven Ionen, um eine elektrostatische Vernetzung durchzuführen, wobei die vernetzte Hyaluronsäure mit positiven Ionen ferner eine durch Polyaminvernetzung erhaltene vernetzte Hyaluronsäure ist, und wobei die Masse des Polyaminvernetzungsmittels 0,5 bis 20 % der Gesamtmasse der Hyaluronsäure ausmacht,
und ferner das Durchführen der Oberflächenladungsmodifikation an den zweiphasigen Mikrokügelchen aufweist: Einweichen der zweiphasigen Mikrokügelchen in einer alkalischen Pufferlösung und Erhalten der modifizierten zweiphasigen Mikrokügelchen durch Abtrennung.

13. Herstellungsverfahren gemäß Anspruch 7, 8 oder 12, wobei ein Massenverhältnis der zweiphasigen Mikrokügelchen zu der vernetzten Hyaluronsäure bei der physikalischen elektrostatischen Vernetzung 1:1-50 und vorzugsweise 1:1-20 beträgt, und
ferner die alkalische Pufferlösung eine alkalische Phosphatpufferlösung ist und der pH-Wert der alkalischen Phosphatpufferlösung vorzugsweise 9,0 bis 11,0 beträgt.

14. Anwendung des Verbundgels gemäß irgendeinem der Ansprüche 1 bis 5 oder des durch das Herstellungsverfahren gemäß irgendeinem der Ansprüche 6 bis 13 hergestellten Verbundgels zur Herstellung von Arzneimittelträgern, Füllstoffen oder Gewebereparaturmaterialien.

**Revendications**

1. Gel composite, préparé par réticulation de microsphères biphasiques avec de l'acide hyaluronique, dans lequel les microsphères biphasiques sont des microsphères biphasiques d'acide polylactique/matériau inorganique ;

la réticulation comprend la réticulation d'acide polylactique dans les microsphères biphasiques avec l'acide hyaluronique ;
l'acide polylactique est de taille micrométrique ;
le matériau inorganique est choisi parmi un ou plusieurs de carbonate de calcium, phosphate de calcium et tartrate de calcium ;
le matériau inorganique est un matériau inorganique nanométrique.

2. Gel composite selon la revendication 1, dans lequel la réticulation de l'acide polylactique avec l'acide hyaluronique comprend l'une quelconque d'une réticulation électrostatique physique et d'une réticulation chimique.

3. Gel composite selon la revendication 1 ou 2, dans lequel le phosphate de calcium est l'hydroxyapatite.

4. Gel composite selon l'une quelconque des revendications 1 à 3, dans lequel l'acide hyaluronique est l'acide hyaluronique réticulé ; en outre, l'acide hyaluronique réticulé est préparé à l'aide d'un agent de réticulation, et l'agent de réticulation est choisi dans un ou plusieurs parmi : dialdéhyde, disulfure, agent de réticulation polyéthylène glycol (PEG), divinylsulfone, éther diglycidique, diépoxyde et polyamine ; et en outre, l'agent de réticulation est une polyamine endogène.

**5.** Gel composite selon l'une quelconque des revendications 1 à 4, dans lequel le gel composite est préparé par réticulation d'acide hyaluronique avec des microsphères biphasiques d'acide polylactique dans lesquelles de l'hydroxyapatite à l'échelle nanométrique est dispersée, la masse de l'hydroxyapatite représentant 0,1 à 50 % de la masse totale des microsphères biphasiques d'acide polylactique dispersées dans l'hydroxyapatite.

**6.** Procédé de préparation d'un gel composite selon l'une quelconque des revendications 1 à 5, comprenant la réticulation de microsphères biphasiques avec l'acide hyaluronique pour former un gel composite, dans lequel les microsphères biphasiques sont des microsphères biphasiques d'acide polylactique/matériau inorganique, et la réticulation comprend la réticulation d'acide polylactique dans les microsphères biphasiques avec l'acide hyaluronique.

**7.** Procédé de préparation selon la revendication 6, dans lequel la réticulation est une réticulation en phase solide ; et en outre, la réticulation en phase solide comprend l'une quelconque parmi une réticulation électrostatique physique et une réticulation chimique.

**8.** Procédé de préparation selon la revendication 6 ou 7, dans lequel l'acide hyaluronique est l'acide hyaluronique réticulé ; l'acide hyaluronique réticulé est préparé à l'aide d'un agent de réticulation ; et l'agent de réticulation est choisi dans un ou plusieurs parmi : dialdéhyde, disulfure, un agent de réticulation de polyéthylène glycol (PEG), divinylsulfone, éther diglycidylique, diépoxyde et polyamine.

**9.** Procédé de préparation selon l'une quelconque des revendications 6 à 8, dans lequel la réticulation chimique comprend la réalisation d'une modification amino sur l'acide polylactique dans les microsphères biphasiques, puis la réaction des microsphères biphasiques modifiées avec l'acide hyaluronique pour former une réticulation par liaison amide ; en outre, la modification amino comprend la réaction des microsphères biphasiques avec un composé aminé ; et en outre, la réticulation par liaison amide comprend la réalisation d'une réaction de couplage sur les microsphères biphasiques modifiées et l'acide hyaluronique en présence d'un agent activateur de carbodiimide.

**10.** Procédé de préparation selon la revendication 9, dans lequel le composé aminé contient une polyamine ou un composé polyamino ;

de préférence, la polyamine comprend l'une quelconque parmi une polyamine aliphatique, une polyamine aromatique et une polyamine hétéroatomique ; le composé polyamino comprend l'un quelconque parmi un PEG aminé bis-terminal et un PEG multi-bras aminé terminal ; et
en outre, une quantité ajoutée du composé aminé est de 30 à 200 % de la masse des microsphères biphasiques, et la concentration du composé aminé est de 10 à 1000 mg/mL.

**11.** Procédé de préparation selon l'une quelconque des revendications 7 à 10, dans lequel un rapport massique des microsphères biphasiques à l'acide hyaluronique dans la réticulation chimique est de 1:0,5-20, et est de préférence de 1:1-10 ;

en outre, l'agent activateur de carbodiimide comprend un ou plusieurs parmi 1-éthyl-3-(3-diméthylaminopropyl) carbodiimide, 1-(3-diméthylaminopropyl)-3-éthyl-carbodiimide, 1-cyclohexyl-3-(2-morpholinoéthyl)carbodiimide, 1,3-bis[di(méthoxyméthyl)méthyl]carbodiimide et leurs sels ;
en outre, une quantité ajoutée de l'agent activateur de carbodiimide est de 10-150 % de la masse totale de l'acide hyaluronique ;
en outre, la concentration d'une solution d'acide hyaluronique est de 10-100 mg/mL ; et
en outre, des températures de réaction de la modification amino et de la réticulation de liaison amide sont de 5-60 °C, et un temps de réaction est de 6-24 h.

**12.** Procédé de préparation selon la revendication 7 ou 8, dans lequel la réticulation électrostatique physique comprend la modification de charge superficielle sur les microsphères biphasiques afin d'obtenir des microsphères biphasiques modifiées dont les surfaces portent des ions négatifs, et le mélange des microsphères biphasiques modifiées et de l'acide hyaluronique réticulé portant des ions positifs, de manière à effectuer une réticulation électrostatique ; en outre, l'acide hyaluronique réticulé ayant des ions positifs est un acide hyaluronique réticulé obtenu par réticulation polyamine ; et en outre, la masse de l'agent de réticulation polyamine représente 0,5-20 % de la masse totale de l'acide hyaluronique ;
et en outre, la modification de charge superficielle sur les microsphères biphasiques comprend le trempage des microsphères biphasiques dans une solution tampon alcaline et l'obtention des microsphères biphasiques modifiées par séparation.

13. Procédé de préparation selon la revendication 7, 8 ou 12, dans lequel un rapport massique des microsphères biphasiques à l'acide hyaluronique réticulé dans la réticulation électrostatique physique est de 1:1-50, et est de préférence de 1:1-20 ; et
en outre, la solution tampon alcaline est une solution tampon phosphate alcaline, et le pH de la solution tampon phosphate alcaline est de préférence de 9,0 à 11,0.

14. Application du gel composite selon l'une quelconque des revendications 1 à 5 ou du gel composite préparé par le procédé de préparation selon l'une quelconque des revendications 6 à 13 dans la préparation de supports de médicaments, de charges ou de matériaux de réparation tissulaire.

Fig. 1

Fig. 2

S4800 3.0kV 8.1mm x100 SE(M)                    500μm

Fig. 3

| 10µm | EHT = 1.00 kV | Signal A = SE2 |
| | WD = 6.3 mm | Mag = 1.00 K X |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 106139256 A
- CN 111905151 A
- CN 101590388 A
- CN 103868658 B
- CN 105797215 A
- CN 109749119 A
- CN 107519536 A
- CN 109350768 A
- CN 102489231 B
- CN 109621003 A
- CN 99813143
- US 9907739 B
- CN 202111326226

### Non-patent literature cited in the description

- **HUANG ZHIHUAN**. *Journal of Composite Materials*, 2021, vol. 38, 749-760
- **DONG YAN**. *Additive Manufacturing*, 2020, vol. 34, 101305